# EUROPÄISCHE PATENTSCHRIFT

(11) **EP 0 416 158 B1**
(45) Veröffentlichungstag und Bekanntmachung des Hinweises auf die Patenterteilung: **30.11.1994**
(21) Anmeldenummer: 89116573.0
(22) Anmeldetag: 07.09.1989
(51) Int. Cl.: A61B 17/39

(54) **Verfahren und Schaltungsanordnung zur Überwachung von mehreren Elektrodenflächen der neutralen Elektrode eines H.F.-Chirurgiegerätes**
Method and circuit for controlling an indifferent electrode with multiple contact surfaces used in HF surgery
Procédé et circuit de contrôle d'une électrode de retour à surfaces de contact multiples pour un appareil de chirurgie à haute fréquence

(43) Veröffentlichungstag der Anmeldung: 13.03.1991
(73) Patentinhaber: SIEMENS AKTIENGESELLSCHAFT, 80333 München (DE)
(72) Erfinder: Künecke, Peter, Dipl.-Ing., D-8520 Buckenhof (DE); Hagen, Uwe, Dipl.-Ing., D-8550 Forchheim (DE)

(56) Entgegenhaltungen:
- DE-A- 3 544 443

## Beschreibung

Die Erfindung bezieht sich auf ein Verfahren zur Überwachung mehrerer Elektrodenflächen der neutralen Elektrode eines HF-Chirurgiegerätes auf flächiges Anliegen am Körpergewebe eines Patienten. Die Erfindung bezieht sich weiterhin auf eine Schaltungsanordnung zur Durchführung des Verfahrens.

Bei bekannten Verfahren und Schaltungsanordnungen der eingangs genannten Art (z.B. gemäß DE-A-3 544 443) wird ein Quellenstrom (Hilfsstrom) auf eine Elektrodenfläche (Hilfselektrode) gegeben, die auf Patienten-Körpergewebe elektrisch leitend kontaktiert ist. An zwei anderen Elektrodenflächen, die ebenfalls mit Patienten-Körpergewebe elektrisch leitend kontaktiert sind, wird der resultierende Strom gemessen. Überschreitet einer der gemessenen Ströme eine zulässige Abweichung zu höheren oder insbesondere zu niedrigeren Strömen im Vergleich zu der anderen Elektrode, wird ein Alarmsignal insbesondere zum selbsttätigen Abschalten eines Elektrotherapievorganges gebildet. Die Abweichung der resultierenden Meßströme entsteht unter anderem durch Ablösung einer Elektrodenfläche vom Patienten. Lösen sich dagegen zwei Elektrodenflächen gleichzeitig so ab, daß die kontaktierten Restflächen, an denen die resultierenden Ströme gemessen werden, annähernd flächengleich sind, weisen die resultierenden Ströme keine auswertbaren Unterschiede auf.

Um die Gefahr der Bildung von gleichgroßen Restflächen zur vermindern, hat man schon die Anzahl der Elektrodenflächen erhöht und/oder den Elektrodenflächen eine besondere Form gegeben. Nachteilig ist aber nach wie vor, daß bei bekannten Verfahren und Schaltungsanordnungen die Fläche der Hilfselektrode nicht in die Überwachung auf teilweise Ablösung einbezogen werden kann.

Der Erfindung liegt die Aufgabe zugrunde, ein Verfahren und eine Schaltungsanordnung der eingangs genannten Art unter Verwendung einer Hilfselektrode anzugeben, womit auch eine Überwachung der Hilfselektrode auf teilweise Ablösung vom Patienten-Körpergewebe ermöglicht wird.

Diese Aufgabe wird durch die im Anspruch 1 bzw. im Anspruch 7 genannten Merkmale gelöst. Durch die Einbeziehung der Hilfselektrode in die Überwachung auf teilweise Ablösung einer oder mehrerer Elektrodenflächen vom Patienten wird der wesentliche Vorteil einer deutlich erhöhten Sicherheit gegenüber Ablösung von Elektrodenflächen mit daraus entstehenden unerwünschten Nebenwirkungen erreicht, zu denen z.B. Verbrennungen insbesondere während einem HF-chirurgischen Eingriff gehören.

Weitere Vorteile und Einzelheiten ergeben sich aus der nachfolgenden Beschreibung von Ausführungsbeispielen anhand der Zeichnungen und in Verbindung mit den Ansprüchen.

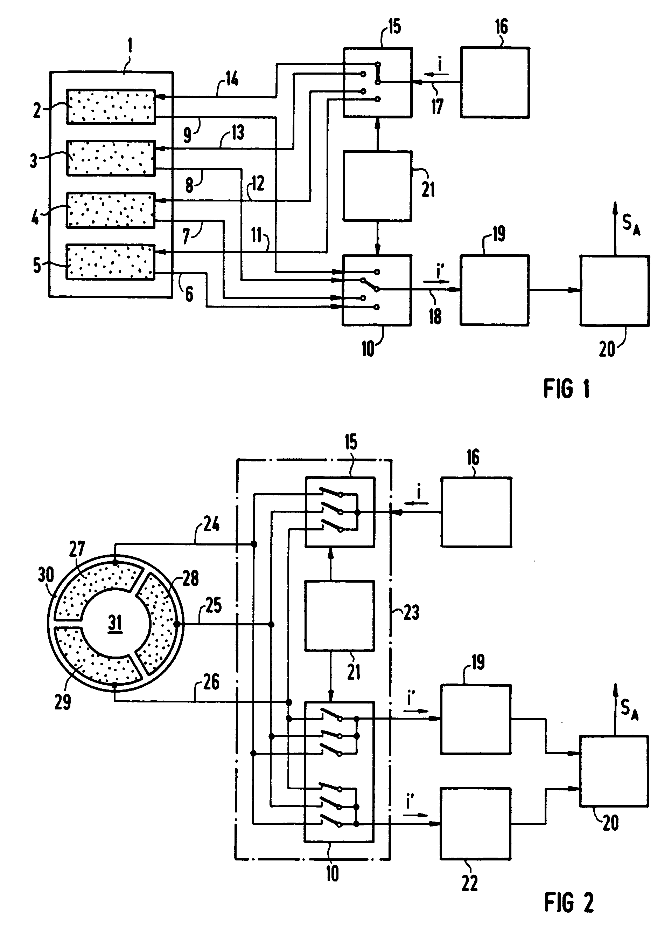

Die Figuren 1 und 2 zeigen in vereinfachten Blockschaltbildern Ausgestaltungen und Varianten dem erfindungsgemäßen Schaltungsanordnung, mit der das erfindungsgemäße Verfahren durchführbar ist.

In Figur 1 sind auf einem gemeinsamen Träger 1 vier Elektrodenflächen 2 bis 5 angeordnet, die über elektrische Leitungen 6 bis 9 mit einer ersten Umschalteinrichtung 10 und über elektrische Leitungen 11 bis 14 mit einer zweiten Umschalteinrichtung 15 verbunden sind. Aus einer Stromquelle 16 wird ein vorzugsweise hochfrequenter Wechselstrom i - dessen ständig wechselnde Stromflußrichtung zur Vereinfachung in nur einer Stromrichtung dargestellt und nachfolgend wie ein Gleichstrom beschrieben wird - über eine Leitung 17 sowie über die zweite Umschalteinrichtung 15 und über die Leitung 14 der Elektrodenfläche 2 zugeführt. Wenn alle Elektrodenflächen 2 bis 5 elektrisch kontaktgebend auf Körpergewebe eines Patienten aufliegen und wenigstens jeweils eine der dann zu überwachenden Elektrodenflächen 3 bis 5, z.B. wie dargestellt die Elektrodenfläche 3, über die erste Umschalteinrichtung 10 und über eine Leitung 18 mit einer Meßeinrichtung 19 verbunden ist, können die jeweils resultierenden Ströme i′ gemessen werden. Dabei sind nicht dargestellte zweite Pole an der Meßeinrichtung 19 und an der Stromquelle 16 zur Bildung eines geschlossenen Stromkreises selbstverständlich miteinander verbunden.

Der in der Meßeinrichtung 19 gemessene Meßwert des resultierenden Stromes i′ wird einer Auswerteeinrichtung 20 zugeführt und dort in einem nicht dargestellten Speicher gespeichert. Anschließend schaltet eine elektronische Steuerung 21 die erste Umschalteinrichtung 10 auf die Leitung 7, die mit der Elektrodenfläche 4 verbunden ist. Dadurch kann ein weiterer resultierender Strom i′ mit der Meßeinrichtung 19 gemessen und das Meßergebnis der Auswerteeinrichtung 20 zugeführt werden. In der Auswerteeinrichtung 20 wird der zuvor gespeicherte Meßwert mit dem jetzt vorliegenden Meßwert auf Gleichheit geprüft. Bei Überschreiten einer zulässigen Abweichung der beiden Meßwerte wird ein Alarmsignal S_{A} gebildet, das zur Steuerung einer nicht dargestellten Alarm- und/oder Abschalteinrichtung zur Unterbrechung des Therapievorganges benutzt werden kann. Anschließend wird in der Auswerteeinrichtung 20 der bisher gespeicherte Meßwert aus der Elektrodenfläche 3 gelöscht und der Meßwert aus der Elektrodenfläche 4 gespeichert. Sodann schaltet die elektronische Steuerung 21 die erste Umschalteinrichtung 10 auf die mit der Elektrodenfläche 5 verbundene Leitung 6, wodurch der zuvor beschriebene Meß-, Prüfungs- und Speicherungsablauf wiederholt wird. Diese gleichartigen Meß- und Prüffolgen bilden einen ersten Verfahrensabschnitt, der es erlaubt, anhand der Meßergebnisse im Falle von Abweichungen Kontaktflächenungleichheiten an den Elektrodenflächen 3 und 4 und an den Elektrodenflächen 4 und 5 festzustellen.

Nach Abschluß dieses ersten Verfahrensabschnittes wird in einem zweiten Verfahrensabschnitt gemäß der Erfindung der Quellenstrom i aus der Stromquelle 16 über die von der elektronischen Steuerung 21 gesteuerte zweite Umschalteinrichtung 15 auf die Leitung 13 geschaltet, die mit der Elektrodenfläche 3 verbunden ist. Dadurch wird der Quellenstrom i erfindungsgemäß in eine zweite Elektrodenfläche 3 von insgesamt vier Elektrodenflächen 2 bis 5 eingespeist. In diesem beschriebenen, jedoch nicht gezeichneten Schaltzustand bildet die Elektrodenfläche 2, die im ersten Verfahrensabschnitt die gespeiste, also erste Elektrodenfläche war, zusammen mit den Elektrodenflächen 4 und 5 zu überwachende Elektrodenflächen. Wird nun der erste Verfahrensabschnitt mit Messung der resultierenden Stromwerte an den nunmehr als zu überwachende Elektrodenflächen 2, 4 und 5 geschalteten Elektrodenflächen wiederholt und wird dabei z.B. ein Überschreiten einer zulässigen Abweichung des Meßwertes an der Elektrodenfläche 2 mit einem vorhergehenden Meßwert an der Elektrode 5 oder mit einem nachfolgenden Meßwert an der Elektrodenfläche 4 festgestellt, wird das Alarmsignal S_{A} ausgelöst. Damit ist die im ersten Verfahrensabschnitt als Hilfselektrode benutzte Elektrodenfläche 2 nunmehr in einem zweiten Verfahrensabschnitt mitüberwacht worden.

Im Ausführungsbeispiel gemäß Figur 1 ist vorgesehen, daß alle Elektroden 2 bis 5 nacheinander über die zweite Umschalteinrichtung 15 als Hilfselektrode in einem Meßzyklus benutzt werden. Es ist aber auch möglich, die zweite Umschalteinrichtung 15 lediglich mit zwei der Elektroden flächen 2 bis 5 umschaltbar zu verbinden, wodurch bereits das Ziel der Erfindung nach einer erhöhten Sicherheit durch Überwachung der jeweiligen Hilfselektrode erreicht werden kann. Der erste und der zweite Verfahrensabschnitt bilden damit schon einen Meßzyklus, der ständig wiederholt werden kann.

Die Meßeinrichtung 19 ist vorzugsweise eine Impedanzmeßeinrichtung, mit der eine Betrags- und Phasenwinkelmessung der jeweils resultierenden Ströme i′ meßbar ist, wodurch die jeweilige Impedanz des Körpergewebes des Patienten zwischen zwei über die Umschalteinrichtungen 10 und 15 kontaktierte Elektrodenflächen 2 bis 5 gemessen werden kann.

Ein Ausführungsbeispiel gemäß Figur 2 unterscheidet sich vom vorhergehenden Ausführungsbeispiel zunächst durch zwei gleichartige Meßeinrichtungen 19 und 22, die mit der Auswerteeinrichtung 20 verbunden sind, die nunmehr keine Speichereinrichtung für ein vorausgegangenes Meßergebnis enthält, weil die beiden Meßeinrichtungen 19 und 22 gleichzeitig jeweils ein Meßergebnis liefern. Des weiteren sind die erste Umschalteinrichtung 10, die beide Meßeinrichtungen 19 und 22 gleichzeitig umschaltet, die elektronische Steuerung 21 und die zweite Umschalteinrichtung 15 in einer Baueinheit 23 vereinigt, von der nur jeweils eine elektrische Leitung 24 bis 26 zu jeweils einer von insgesamt drei Elektrodenflächen 27 bis 29 führt. Die gegeneinander isolierten und auf einem gemeinsamen Träger 30 angeordneten Elektrodenflächen 27 bis 29 sind als drei Abschnitte einer gemeinsamen Kreisfläche ausgebildet, die eine von Elektrodenflächen freie Kreisfläche 31 umschließt. Durch diese zueinander symmetrische Anordnung der Elektrodenflächen 27 bis 29 kann die zulässige Abweichung der Meßwerte gering gehalten und damit von vornherein die Patientensicherheit noch weiter erhöht werden.

In der gezeichneten Darstellung gemäß Figur 2 ist die Stromquelle 16 über die zweite Umschalteinrichtung 15 und die Leitung 24 mit der Elektrodenfläche 27 verbunden, die im ersten Verfahrensabschnitt eine Hilfselektrode bildet. Die beiden anderen Elektrodenflächen 28 und 29 sind über die erste Umschalteinrichtung 10 mit je einer Meßeinrichtung 19 bzw. 22 verbunden.

Nach Auswertung der beiden, zugleich vorliegenden Meßergebnisse in der Auswerteeinrichtung 20 schaltet die elektronische Steuerung 21 die Stromquelle 16 über die zweite Umschalteinrichtung 15 und über die Leitung 25 auf die Elektrodenfläche 28. Zugleich schaltet die elektronische Steuerung 21 die beiden Elektrodenflächen 27 und 29 über die jeweils zugehörigen Leitungen 24 bzw. 26 mit der ersten Umschalteinrichtung 10 auf je eine Meßeinrichtung 19 bzw. 22. In diesem zweiten Verfahrensabschnitt ist im Gegensatz zum ersten dargestellten Verfahrensabschnitt nunmehr die Elektrodenfläche 28 als Hilfselektrode geschaltet. Die im ersten Verfahrensabschnitt als Hilfselektrode geschaltete Elektrodenfläche 27 und die andere Elektrodenfläche 29 sind im jetzt beschriebenen zweiten Verfahrensabschnitt zu überwachende Elektrodenflächen. Der erste und der zweite Verfahrensabschnitt bilden einen Meßzyklus, der wiederholt werden kann, sobald die Meßergebnisse aus den beiden Meßeinrichtungen 19 und 22 in der Auswerteeinrichtung 20 ausgewertet wurden. Es könnte sodann die elektronische Steuerung 21 die erste Umschalteinrichtung 10 und die zweite Umschalteinrichtung 15 wieder in die dargestellte Schaltstellung zurückschalten.

Die Überwachungsmöglichkeiten und damit die Patientensicherheit läßt sich noch steigern, wenn in einem weiteren (dritten) Verfahrensabschnitt die dritte Elektrodenfläche 29 über die zweite Umschalteinrichtung 15 mit der Stromquelle 16 mittels der elektronischen Steuerung 21 verbunden wird. In diesem dritten Verfahrensabschnitt werden dann die Elektrodenflächen 27 über die Leitung 24 und über die erste Umschalteinrichtung 10 mit der Meßeinrichtung 19 verbunden. Des weiteren wird gleichzeitig die Elektrodenfläche 28 über die Leitung 25 und über die erste Umschalteinrichtung 10 mit der Meßeinrichtung 22 verbunden. Nach Auswertung der beiden gleichzeitig vorliegenden Meßergebnisse aus den beiden Meßeinrichtungen 19 und 22 in der einen Auswerteeinrichtung 20 ist auch dieser dritte Verfahrensabschnitt abgeschlossen, der mit den beiden vorausgehenden Verfahrensabschnitten einen Meßzyklus bildet. Die elektronische Steuerung 21 schaltet nach Abschluß dieses ersten Meßzyklusses die erste Umschalteinrichtung 10 und die zweite Umschalteinrichtung 15 wieder in die dargestellte Schaltstellung, wodurch ein zweiter Meßzyklus eingeleitet wird, der dem ersten Meßzyklus ablaufmäßig entspricht.

## Patentansprüche

1. Verfahren zur Überwachung mehrerer Elektrodenflächen der neutralen Elektrode eines HF-Chirurgiegerätes auf flächiges Anliegen am Körpergewebe eines Patienten, gekennzeichnet durch die Verfahrensschritte
- in eine erste Elektrodenfläche (2; 27) wird ein Quellenstrom (i) eingespeist,
- an allen oder einigen der übrigen Elektrodenflächen (3 bis 5; 28, 29) werden die resultierenden Stromwerte (i′) gemessen,
- wenigstens jeweils zwei Meßwerte werden auf Gleichheit geprüft,
- bei Überschreiten einer zulässigen Abweichung der Meßwerte wird ein Alarmsignal (S_{A}) gebildet,
- in einem zweiten Verfahrensabschnitt wird der Quellenstrom (i) in eine zweite Elektrodenfläche (3; 28) eingespeist, an allen oder einigen der übrigen, die erste Elektrodenfläche (2; 27) jetzt einschließenden Elektrodenflächen (2,4,5; 27, 29) werden wieder die resultierenden Stromwerte (i′) gemessen wenigstens jeweils zwei Meßwerte werden wieder auf Gleichheit geprüft und bei Überschreiten der zulässigen Abweichung wird ein Alarmsignal (S_{A}) gebildet,
- weitere Verfahrensabschnitte mit derart rotierender oder wechselseitiger Quellenstromeinspeisung, Stromwertmessung, Meßwertvergleichsbildung und eventueller Alarmsignalbildung schließen sich an.

2. Verfahren nach Anspruch 1, wobei der erste Verfahrensabschnitt und wenigstens einer der weiteren Verfahrensabschnitte einen Meßzyklus bilden, der wiederholt wird, sobald nacheinander in wenigstens zwei Elektrodenflächen (2, 3; 27, 28) einmal der Quellenstrom (i) eingespeist wurde.

3. Verfahren nach Anspruch 2, wobei ein vorhergehender Meßzyklus wiederholt wird, sobald nacheinander in allen Elektrodenflächen (2 bis 5; 27 bis 29) einmal der Quellenstrom (i) eingespeist wurde, wobei die Reihenfolge der Einspeisung elektronisch vorgegeben ist.

4. Verfahren nach einem der Ansprüche 1 bis 3 wobei an jeweils zwei der zu überwachenden Elektrodenflächen (28, 29) gleichzeitig eine Messung der resultierenden Stromwerte (i′) vorgenommen wird und diese Stromwerte auf Gleichheit geprüft werden.

5. Verfahren nach einem der Ansprüche 1 bis 4, wobei der Quellenstrom (i) ein Wechselstrom ist, mittels einer Betrags- und Phasenwinkelmessung der jeweils resultierenden Ströme (i′) zwischen der eingespeisten Elektrodenfläche (2; 27) und der jeweils überwachten Elektrodenfläche (3 bis 5; 28, 29) die Impedanz des Patienten-Körpergewebes bestimmt und auf Gleichheit geprüft sowie bei Überschreiten einer zulässigen Abweichung der Impedanzwerte das Alarmsignal (S_{A}) gebildet wird.

6. Schaltungsanordnung zur Durchführung des Verfahrens nach einem der Ansprüche 1 bis 5, bei der wenigstens eine Meßeinrichtung (19, 22), die ausgangsseitig mit einer Auswerteeinrichtung (20) verbunden ist, eingangsseitig über eine erste Umschalteinrichtung (10) mit wenigstens jeweils einer zu überwachenden Elektrodenfläche (2 bis 5; 27 bis 29) zeitlich aufeinanderfolgend elektrisch verbindbar ist und bei der eine Stromquelle (16) für Meßzwecke über eine zweite Umschalteinrichtung (15) zeitlich aufeinanderfolgend mit wenigstens zwei anderen Elektrodenflächen (2, 3; 27, 28) elektrisch verbindbar ist, die nicht gleichzeitig auf die Meßeinrichtung (19, 20) geschaltet sind.

7. Schaltungsanordnung nach Anspruch 6, bei der die erste und zweite Umschalteinrichtung (10 bzw. 15) von einer gemeinsamen elektronischen Steuerung (21) geschaltet wird.

8. Schaltungsanordnung nach Anspruch 6 oder 7, bei der die erste und zweite Umschalteinrichtung (10 bzw. 15) in einer Baueinheit (23) vereinigt sind, von der nur jeweils eine elektrische Leitung (24 bis 26) zu jeder Elektrodenfläche (27 bis 29) führt.

9. Schaltungsanordnung nach einem der Ansprüche 6 bis 8, bei der die Meßeinrichtung (19, 22) eine Impedanzmeßeinrichtung ist, die die Impedanz des Patienten-Körpergewebes zwischen der jeweils eingespeisten Elektrodenfläche (2; 27) und der mit der Impedanzmeßeinrichtung jeweils verbundenen Elektrodenfläche (3; 28,29) mißt.

10. Schaltungsanordnung nach einem der Ansprüche 6 bis 9, bei der über die erste Umschalteinrichtung (10) zwei Meßeinrichtungen (19, 22) gleichzeitig mit jeweils zwei zu überwachenden Elektrodenflächen (28, 29) zeitlich aufeinanderfolgend verbindbar sind.

11. Schaltungsanordnung nach einem der Ansprüche 6 bis 10, bei der die Elektrodenflächen (27 bis 29) gegeneinander isolierte Teilflächen einer dreiteiligen Elektrode sind, deren Teilflächen Abschnitte einer gemeinsamen Kreisringfläche sind, die eine von den Elektrodenflächen freie Kreisfläche (31) auf einem gemeinsamen Träger (20) umschließt.

## Claims

1. A method of monitoring a plurality of electrode surfaces of the neutral electrode of a HF surgery device to determine the planar adjacency of said surfaces against the body tissue of a patient, characterised by the following steps:
- a source current (i) is fed into a first electrode surface (2; 27),
- the resultant current values (i') are measured at all of or some of the other electrode surfaces (3 to 5; 28, 29),
- in each case at least two measured values are checked for identity,
- in the event of the overshooting of a permissible deviation of the measured values, an alarm signal (S_{A}) is formed,
- in a second stage of the method the source current (i) is fed into a second electrode surface (3; 28), the resultant current values (i') are measured again at all of or some of the other electrode surfaces (2, 4, 5; 27, 29) which now include the first electrode surface (2; 27), in each case at least two measured values are checked again for identity and in the event of the overshooting of the permissible deviation an alarm signal (S_{A}) is formed,
- there follow further stages of the method with such rotary or alternate feeding-in of the source current, current value measurement, measured value comparison formation and possible alarm signal formation.

2. A method as claimed in Claim 1, wherein the first stage of the method and at least one of the further stages of the method form a measuring cycle which is repeated as soon as the source current (i) has been fed successively into at least two electrode surfaces (2, 3; 27, 28).

3. A method as claimed in Claim 2, wherein a preceding measuring cycle is repeated as soon as the source current (i) has been fed successively into all the electrode surfaces (2 to 5; 27 to 29), where the sequence of the feeding-in is electronically predetermined.

4. A method as claimed in one of Claims 1 to 3, wherein a measurement of the resultant current values (i') is carried out simultaneously at in each case two of the electrode surfaces (28, 29) to be monitored and these current values are checked for identity.

5. A method as claimed in one of Claims 1 to 4, wherein the source current (i) is an alternating current, by means of a measurement of the magnitude and phase angle of the respective resultant currents (i') between the fed electrode surface (2; 27) and the respective monitored electrode surface (3 to 5; 28, 29) the impedance of the body tissue of the patient is determined and checked for identity, and in the event of the overshooting of a permissible deviation of the impedance values the alarm signal (S_{A}) is formed.

6. A circuit arrangement for the implementation of the method as claimed in one of Claims 1 to 5, wherein at least one measuring device (19, 22), which is connected at its output end to an analysis device (20), can be successively electrically connected at its input end via a first switch-over device (10) in each case to at least one electrode surface (2 to 5; 27 to 29) to be monitored, and wherein for measurement purposes a current source (16) can be successively electrically connected via a second switch-over device (15) to at least two other electrode surfaces (2, 3; 27, 28) which are not simultaneously connected to the measuring device (19, 20).

7. A circuit arrangement as claimed in Claim 6, wherein the first and second switch-over devices (10, 15) are switched by a common electronic control unit (21).

8. A circuit arrangement as claimed in Claim 6 or 7, wherein the first and second switch-over devices (10 and 15) are combined in one structural unit (23) from which only one respective electric line (24 to 26) leads to each electrode surface (27 to 29).

9. A circuit arrangement as claimed in one of Claims 6 to 8, wherein the measuring device (19, 22) is an impedance measuring device which measures the impedance of the body tissue of the patient between the respective fed electrode surface (2; 27) and the respective electrode surface (3; 28, 29) connected to the impedance measuring device.

10. A circuit arrangement as claimed in one of Claims 6 to 9, wherein via the first switch-over device (10) two measuring devices (19, 22) simultaneously can be connected successively to two respective electrode surfaces (28, 29) to be monitored.

11. A circuit arrangement as claimed in one of Claims 6 to 10, wherein the electrode surfaces (27 to 29) are mutually insulated partial surfaces of a three-part electrode, the partial surfaces of which form sections of a common circular ring-shaped surface which encloses a circular surface (31), free of the electrode surfaces, on a common carrier (20).

## Revendications

1. Procédé pour vérifier si plusieurs surfaces de l'électrode neutre d'un appareil chirurgical à haute fréquence sont appliquées à plat sur le tissu corporel d'un patient, caractérisé par les étapes opératoires suivantes:
- un courant de source (i) est injecté dans une première surface (2;27) de l'électrode,
- les intensités de courant résultantes (i') sont mesurées sur toutes ou sur quelques-unes des autres surfaces (3 à 5; 28,29) de l'électrode,
- au moins deux valeurs de mesure respectives sont contrôlées pour déterminer si elles sont égales,
- lors du dépassement d'un écart admissible des valeurs de mesure, un signal d'alarme (S_{A}) est formé,
- lors d'une seconde étape opératoire, le courant de source (i) est injecté dans une seconde surface (3; 28) de l'électrode, et à nouveau les intensités de courant résultantes (i') sont mesurées sur toutes ou sur quelques-unes des autres surfaces (2,3,4,5,27,29) de l'électrode, qui incluent maintenant la première surface (2;27) de l'électrode, au moins respectivement deux valeurs de mesure sont à nouveau contrôlées pour déterminer si elles sont égales et, lors du dépassement de l'écart admissible, un signal d'alarme (S_{A}) est formé,
- d'autres étapes opératoires avec la mise en oeuvre de manière cyclique ou alternativement, d'une injection du courant de source, d'une mesure de l'intensité, du courant, d'une comparaison de valeurs de mesure et d'une formation éventuelle d'un signal d'alarme sont ensuite mises en oeuvre.

2. Procédé suivant la revendication 1, selon lequel la première étape opératoire et au moins l'une des autres étapes opératoires forment un cycle de mesure, qui est répété dès que le courant de source (i) a été injecté successivement dans au moins deux surfaces (2,3;27,28) de l'électrode.

3. Procédé suivant la revendication 2, selon lequel un cycle de mesure précédent est répété dès que le courant de source (i) a été injecté successivement dans toutes les surfaces (2 à 5; 27 à 29) de l'électrode, la succession de l'injection étant prédéterminée électroniquement.

4. Procédé suivant l'une des revendications 1 à 3, selon lequel une mesure des valeurs d'intensité résultantes (i') est réalisée au niveau de respectivement deux surfaces à contrôler (28,29) de l'électrode, et ces valeurs d'intensité sont contrôlées pour déterminer si elles sont égales.

5. Procédé suivant l'une des revendications 1 à 4, selon lequel le courant de source (i) est un courant alternatif, l'impédance du tissu corporel du patient est déterminée au moyen d'une mesure de la valeur absolue et de l'angle de phase des courants respectivement résultant (i') entre la surface (2;24) de l'électrode, dans laquelle l'injection est réalisée, et la surface respectivement contrôlée (3 à 5; 28,29) de l'électrode, et est contrôlée pour déterminer si elle est identique, et le signal d'alarme (S_{A}) est formé dans le cas du dépassement d'un écart admissible des valeurs d'impédance.

6. Montage pour la mise en oeuvre du procédé suivant l'une des revendications 1 à 5, dans lequel au moins un dispositif de mesure (19,22), qui est relié côté sortie à un dispositif d'exploitation (20), peut être raccordé électriquement côté entrée, par l'intermédiaire d'un premier dispositif de commutation (10), successivement dans le temps à au moins respectivement une surface à contrôler (2 à 5; 27 à 29) de l'électrode, et dans lequel une source de courant (16) peut être raccordée électriquement, à des fins de mesure, par l'intermédiaire d'un second dispositif de commutation (15), d'une manière successive dans le temps à au moins deux autres surfaces (2,3;27,28) de l'électrode, qui ne sont pas raccordées simultanément au dispositif de mesure (19,20).

7. Montage suivant la revendication 6, dans lequel les premier et second dispositifs de commutation (10 ou 15), sont activés par une unité électronique de commande commune (21).

8. Montage suivant la revendication 6 ou 7, dans lequel les premier et second dispositifs de commutation (10 ou 15) sont réunis dans une unité de construction (23), dont seule respectivement une ligne électrique (24 ou 26) mène à chaque surface (27 à 29) de l'électrode.

9. Montage suivant l'une des revendications 6 à 8, dans lequel le dispositif de mesure (19,22) est un dispositif de mesure d'impédance, qui mesure l'impédance du tissu corporel du patient entre la surface (2;27) de l'électrode, dans laquelle est respectivement réalisée l'injection et la surface (3;28,29) de l'électrode reliée respectivement au dispositif de mesure d'impédance.

10. Montage suivant l'une des revendications 6 à 9, dans lequel les dispositifs de mesure (19,22) peuvent être reliés simultanément, par l'intermédiaire du premier dispositif de commutation (10), successivement dans le temps à respectivement deux surfaces à contrôler (28,29) de l'électrode.

11. Montage suivant l'une des revendications 6 à 10, dans lequel les surfaces (27 à 29) de l'électrode sont des surfaces partielles isolées l'une de l'autre d'une électrode en trois parties, dont les surfaces partielles sont des éléments d'une surface commune en forme d'anneau circulaire, qui entoure une surface circulaire (31), ne contenant pas les surfaces de l'électrode, sur un support commun (20).
